# EUROPEAN PATENT APPLICATION

(11) **EP 4 800 696 A1**
(43) Date of publication of application: **02.09.2026**
(21) Application number: 25160336.1
(22) Date of filing: 26.02.2025
(51) Int. Cl.: G16H 20/17, A61B 5/145, G06N 3/006, G06N 3/045, G06N 3/08, G06N 3/09, G06N 3/092, G16H 50/50, G16H 50/70, A61M 5/14

(54) **COMPUTER IMPLEMENTED METHOD OF DETERMINING AN INSULIN DOSE VALUE FOR A PATIENT NEXT TO ADMINISTER AND TRAINING METHOD THEREFOR**

(71) Applicant: OneTwenty AG, 8046 Zürich (CH)
(72) Inventor: Stiels, Julian Maximilian, 8046 Zürich (CH); Khadhraoui, Mohamed Aous, 8046 Zürich (CH)
(74) Representative: WSL Patentanwälte Partnerschaft mbB

(57) **Abstract**

The present invention relates to a computer implemented training method for training a first artificial intelligence agent for glycemic control of a patient and also relates to a computer implemented method of determining an insulin dose value for a patient next to administer and for administration by an insulin administration device based on a treatment algorithm comprising a first artificial intelligence agent.

## Description

The present invention relates to a computer implemented training method for training a first artificial intelligence agent for glycemic control of a patient and also relates to a computer implemented method of determining an insulin dose value for a patient next to administer and for administration by an insulin pump based on a treatment algorithm comprising a first artificial intelligence agent.

The management of diabetes mellitus presents a significant and ongoing challenge for millions of individuals worldwide. Diabetes, a chronic metabolic disorder, is primarily characterized by the body's inability to regulate blood glucose levels due to either insufficient insulin production (as seen in Type 1 diabetes) or an impaired response to insulin (as seen in Type 2 diabetes). Maintaining blood glucose within a narrow, healthy range is critical to preventing both acute and long-term complications associated with the condition. Hyperglycemia (excessive blood glucose) and hypoglycemia (insufficient blood glucose) each pose distinct and serious health risks, ranging from immediate symptoms like confusion, dizziness, and unconsciousness to long-term damage affecting vital organs such as the heart, kidneys, eyes, and nerves.

To mitigate these risks, individuals with diabetes often rely on external insulin administration, either through manual injections or with the aid of an insulin administration device. Insulin pumps or pens are medical devices designed to deliver precise, controlled doses of insulin throughout the day, thereby assisting in the regulation of blood glucose level. Insulin pumps offer continuous infusion and, in some cases, automated adjustments based on sensor feedback. Smart pens may be used for repeated manual injection based on an automatic insulin dose calculation, setting and/or control. Despite these advancements, the process of maintaining glucose homeostasis remains complex and burdensome for users.

The principal challenge lies in the dynamic nature of blood glucose fluctuations, which are influenced by a variety of unpredictable factors such as meal composition, physical activity, stress, illness, and individual metabolic variability. Users must constantly monitor their blood glucose level, interpret the data, and make dosing decisions in real time. These tasks demand vigilance, experience, and a deep understanding of the body's physiological responses - a cognitive burden that can result in human error. Mistakes in dosing decisions can lead to critical events such as severe hypoglycemia, which may cause loss of consciousness, seizures, or even death, or to persistent hyperglycemia, which increases the risk of long-term complications like cardiovascular disease and neuropathy.

Despite the availability of continuous glucose monitoring (CGM) systems and advancements in sensor technology, the process of determining the optimal insulin dose remains highly dependent on user intervention. While some modern insulin pumps have begun to integrate sensor data for semi-automated insulin delivery, most systems still require manual input from users to confirm dosing decisions. This reliance on human judgment introduces variability and limits the effectiveness of current solutions in achieving stable, near-constant glucose control.

Addressing this problem requires a more sophisticated approach to blood glucose management - one that can anticipate glucose fluctuations, respond in real time, and reduce the cognitive load on users. An ideal solution would provide a fully automated, adaptive insulin dosing system capable of optimizing glycemic control with minimal user intervention. Such a system would significantly improve the quality of life for people with diabetes, reduce the risk of acute complications and related chronic diseases, and lessen the mental and emotional burden associated with managing the diabetes on a daily basis.

Against this background, the underlying problem of the present invention is to provide a computer implemented method facilitating an improved glycemic control of a patient using an insulin administration device.

This problem is solved by a computer implemented training method for training a first artificial intelligence agent for glycemic control of a patient, wherein the training method comprises a basic training session comprising one or more training epochs, wherein a first training data set of real patient data is used as input data for the basic training session, such that in course of the basic training session the first Al agent receives real-patient chronological continuous data sequences and is trained to control the glycemic behavior of the patient based on these real-patient chronological continuous data sequences, wherein the training method comprises additionally a simulator-based training session comprising one or more training epochs, wherein during a training epoch, particularly each training epoch, of the simulator-based training session the first AI agent is trained to control the glycemic behavior of the patient based on glycemic feedback data of simulated patients, wherein the glycemic feedback data is generated by a metabolic simulator.

In the context of the present invention, "to control the glycemic behavior of the patient" means to determine an insulin dose value to be administered based on predictions of future blood glucose values and historic blood glucose values. For example, the first AI agent may determine the expected blood glucose values for several possible insulin dose values and selects the insulin dose value that, if applied, best matches a pre-defined blood glucose range and blood glucose behavior policy (e.g. inhibiting sudden changes in blood glucose levels).

For example, the first simulator-based training session is conducted after the basic training session, such that the agent is already pre-trained by the basic training session when conducting the first simulator-based training session.

For example, the basic training session may be configured that the first AI agent is explicitly not taught by simulator-feedback during the basic training session. Additionally or alternatively, the basic training session may be configured that solely real patient data is used as input data for the basic training session.

In the context of the present invention, an "artificial intelligence agent" (AI agent) is a software-based system that can perceive its environment, process information, and take actions to achieve specific goals or objectives. AI agents operate autonomously, making decisions based on a combination of pre-defined rules, learned behaviors, and real-time inputs from their environment. They are powered by machine learning algorithms, statistical models, or neural networks, which allow them to learn from data and improve their performance over time.

Generally, both the basic training session and the simulator-based training session of the first Al agent are machine learning trainings.

The first AI agent is configured to optimize insulin delivery for enhanced glycemic control of the patient. By analyzing data from continuous glucose monitors (CGMs), user inputs, and historical patterns, the first AI agent predicts future glucose levels and provides insulin dosing accordingly, in particular it provides an insulin dose value next to administer for the respective patient as indicated below. This dynamic decision-making process enables more precise glycemic control, reduces the cognitive burden on users, and enhances the overall effectiveness of automated insulin delivery systems.

By combining a basic training session in which the first AI agent is trained with real patient data and at least one simulator-based training session in which the first AI agent is then confronted with different simulated scenarios and thus further trained, a trained first AI agent is created whose prediction accuracy is more than 50% better than the accuracy of previously known methods for determining insulin dose values.

In the basic training session, the first AI agent is trained to mimic real patient data, which usually includes CGM time sequences and administered insulin data from the respective time period, as closely as possible. This means that the first AI agent is first trained to mimic a therapy - in terms of insulin dose values - that has taken place for one or more patients in reality and resulted in positive glycemic outcomes. This is because the real data comes from patients who have undergone insulin therapy. In contrast, in the simulator-based training session, the AI agent is confronted with multiple simulated scenarios that run through. Accordingly, the first AI agent is tested with different therapies with respect to different scenarios (e.g. different patient types and/or patient situations, regarding, for example, age, sex, stability concerning glycemic response, meal times, sleep time, exercise). In this way, the first AI agent may be trained so that the therapy calculated by the first AI agent - in terms of insulin dose values to be administered - leads to the simulated glycemic behavior - in terms of simulated CGM time sequences - remaining within at least one pre-defined constraint, e.g. within the range from 70 mg/dl to 180 mg/dl. An alternative or second constraint may be formed by a pre-defined minimum CGM value of, for example, 54 mg/dl. A CGM value simulated by the metabolic simulator based on the determined insulin dose value to be administered must not fall below this pre-defined minimum CGM value.

A "training data set of real patient data" refers to a collection of real patient data used to train the first AI agent. This dataset may include information that is directly obtained from real patient data or obtained from real patient data by using standard data pre-processing methods. The training data set of real patient data may include a wide range of information, such as blood glucose measurement data, insulin dosing records, metabolic disturbance data, e.g. carbohydrate intake details, physical activity logs, and other physiological parameters that influence metabolic processes. They may be a continuous data sequence covering one or several pre-defined time period(s) for the respective patient, wherein the corresponding time information is provided, e.g., by an assigned time stamp. Real patient data consists of actual measurements and observations collected from individuals through devices like continuous glucose monitors (CGMs), data of insulin doses actually administered, wearable sensors, or manual input from users/HCP.

In one embodiment, the training data set of real patient data may be filled in / extended by synthesized or simulated patient data, for example if some data are missing. Simulated patient data involves data generated using patient simulators to mimic the behavior of real patients under various conditions. Synthesized patient data includes datasets that are created by processing, augmenting or transforming real patient data, especially in the present context to expand the variety of the training set data for the training of the first AI agent.

In the context of the present invention, "training epochs" refer to the number of complete passes through an entire training dataset during the process of training. Each epoch involves feeding the full dataset into the learning algorithm, allowing the first AI agent to update its internal parameters, such as weights, biases and/or hyperparameters, to increase accuracy in its predictions.

In the context of the present invention, a training session refers to the process of teaching the first Al agent using a set of data, known as the above-mentioned training dataset, to achieve a specific level of accuracy or performance. This session is often composed of multiple training epochs, where an epoch is a complete pass through the entire training dataset.

For example, a basic training session of the first AI agent may comprise between 150 and 250 training epochs. It turned out that this amount of training epochs is sufficient and necessary to derive and a sufficiently pre-trained AI agent.

During each epoch, the first AI agent learns by adjusting its internal parameters (like weights in neural networks) to minimize the error between its predictions and the actual data outcomes, i.e. optimizing by use of a loss function. The goal of each epoch is to improve the first AI agent's accuracy and reduce its loss on the training data. Also, the hyperparameters of the training method can be updated after each training epoch.

A training session ca be defined not just by the completion of its epochs, but by the achievement of a pre-defined convergence criterion. This criterion can be, for example, a predefined threshold of performance that determines when the first AI agent has learned sufficiently from the training data. The convergence criterion could be based on various factors such as a minimum loss value, a maximum accuracy, or a stabilization of the first AI agent's performance across epochs, indicating that further learning would result in negligible improvement.

In such a case, the end of a training session of the first AI agent occurs when this convergence criterion is met, signaling that the first AI agent is optimally trained and further training would likely lead to overfitting, where the first AI agent learns the training data too well, including its noise and errors, which reduces its ability to perform well on unseen data.

In the context of the present invention, a "hyperparameter" is a predefined, customizable configuration variable that controls the learning process of an AI agent, but is not learned by the agent itself during training epochs. Unlike model parameters, which are derived from the training data during the training epochs, hyperparameters are defined before a training epoch begins and can be adapted if needed upon every new training epoch to arrive at a local or global optimum. The hyperparameters have a significant impact on the efficiency, convergence and accuracy of the first AI agent. Hyperparameters determine the structure, behavior and optimization of the learning algorithm. Examples include the learning rate, the batch size, the number of epochs, the number of layers and neurons in a neural network, the activation functions and the regularization parameters. Hyperparameters can be selected using heuristics, manual fine-tuning or automated search methods such as grid search or random search. In the present invention, it is based on data provided with the metabolic simulator and real patient data, in particular data sets of continuous data. The choice of hyperparameters has a decisive influence on the ability of the first AI agent to recognize patterns, avoid overfitting and minimize the overall processing time. In other words, hyperparameters define the learning environment of the first AI agent.

According to an embodiment of the present invention, during the basic training session, a first loss function is used, wherein the first loss function comprises a deviation norm for the deviation between a glycemic behavior prediction of the first AI agent and the glycemic behavior according to the first training data set of real patient data and/or a deviation norm for the deviation between insulin dose values to be administered determined by the first AI agent and insulin dose values administered according to the first training data set of real patient data. It has been shown that this results in a particularly well pre-trained first AI agent whose dosing behavior corresponds to the dosing behavior of real human being, well controlling blood glucose levels.

Additionally or independently, during the simulator-based training session, a second loss function can be used, wherein the second loss function comprises a deviation norm for a deviation between a glycemic behavior driven by insulin does values, which are determined by the first AI agent, and a predefined optimal range of the glycemic behavior of simulated patients.

Hence, the first loss function forms the necessary basis for further training with the second loss function, which in combination results in an agent whose dosing behavior has a quality that significantly exceeds the quality of conventional therapy actors.

According to an embodiment of the present invention, during a training epoch, particularly each training epoch, of the simulator-based training session the first AI agent is confronted with several different simulated glycemic scenarios, which differ, for example, with respect to a simulated patient of the underlying patient type characterized, for example, by its age and sex, wherein for each scenario the following steps are conducted using a metabolic simulator, wherein steps c) and d) are repeated multiple times::
a) The first AI agent receives initial simulated patient data from the metabolic simulator.
b) The first Al agent determines an insulin dose value next to administer based on the initial simulated patient data received in step a).
c) The metabolic simulator determines the simulated patient's glycemic behavior as glycemic feedback data based on the insulin dose value determined in step b) as if the directly previously determined insulin dose value had been administered to the simulated patient.
d) The first Al agent determines an insulin dose value next to administer based on the feedback data determined in step c) and continues with step c) using the determined insulin dose value next to administer in step d).

Steps c and d can be repeated over and over again in sequential simulations, so that the first AI agent simulates the chronological continuous course of a glucose control with the help of the metabolic simulator. This approach significantly increases the quality of dose determination of the first AI agent, which is pre-trained with real patient data in the best case, as the first AI agent can experience and play through different therapy approaches so that it can ultimately learn an optimized therapy approach.

According to an embodiment of the present invention, the metabolic simulator of the simulator-based training session is a data-driven metabolic simulator, wherein the feedback data generated by the data-driven metabolic simulator is determined as the metabolic response in dependence on real or simulated patient data, wherein the data-driven metabolic simulator is collected, e.g. a data-driven grey-box identification simulator like the proprietary ONEsim of the One Twenty AG.

According to an embodiment of the present invention, the metabolic simulator of the simulator-based training session is a pre-defined metabolic simulator, e.g. a non-linear system identification simulator like the T1DMS by UVA/Padova.

In the context of the present invention, a "metabolic simulator" is a computational software system designed to mimic and simulate the physiological processes of human metabolism. It allows for the prediction and analysis of metabolic responses to various inputs, such as carbohydrate load data, physical activity, and medical interventions like insulin administration.

In the context of the present invention, a "pre-defined metabolic simulator" uses solely mathematical models for predicting the glycemic behavior.

In the context of the present invention, a "data-driven metabolic simulator" is a computational software system that utilizes real patient data to model, predict, and simulate the glycemic behavior. Such data-driven metabolic simulators can rely themselves on large datasets and machine learning algorithms to identify patterns, relationships, and behaviors within the system being studied. For example, by leveraging historical and real-time data, these simulators can generate realistic and dynamic simulations that evolve in response to new inputs. By using the data-driven metabolic simulator, the above described "derived or synthesized patient data" can be generated based on real patient data.

According to an embodiment of the present invention, the sets of real patient data comprise Continuous Glucose Monitoring (CGM) data, administered insulin data and data referring to a metabolic disturbance, e.g. carbohydrate load data, each data having a time stamp. In one embodiment, the sets of real patient data comprises physical activity logs.

In the context of the present invention, "Carbohydrate load data" refers to any information pertaining to the amount of carbohydrates that are or will be consumed by an individual. This data may be categorized as announced, estimated, or predicted carbohydrate load data. Announced carbohydrate load data refers to information explicitly provided by the user, such as the carbohydrate content of a planned meal entered into a device or application. Estimated carbohydrate load data refers to approximations of actual or planned carbohydrate intake based on general meal descriptions, portion sizes, or visual assessment of food items. Predicted carbohydrate load data is derived from predictive models or algorithms that infer potential or actual carbohydrate consumption based on historical user behavior, contextual factors, or real-time sensor inputs. Predicted carbohydrate load data may partly or fully replace user-provided carbohydrate load data.

According to an embodiment of the present invention, each original value of the carbohydrate load data of the real or in-silico patient data sets is assessed using the data-driven simulator and corrected based on the result of this assessment determining a respective corrected value of the carbohydrate load data, wherein the respective corrected value is derived from the respective original value by decreasing, increasing or a holding constant depending on the result of the assessment, wherein the corrected values of the carbohydrate load data of the real patient sets are used for hyperparameter adaption instead of the original real patient data values. As indicated above, this may increase accuracy of the first artificial intelligence agent.

According to an embodiment of the present invention, the first AI agent is a reinforcement learning agent, preferably a Twin-Delayed Deep Deterministic Policy Gradient agent, such that the training of the first AI agent is performed by the interaction of an actor, for example an actor neuronal network, and at least one critic, for example a critic neuronal network. It turned out that using such AI agent types gave the best prediction performance and computational efficiency at the same time.

The underlying problem is also solved by a computer implemented method of determining an insulin dose value for a patient next to administer and for administration by an insulin administration device comprising the usage the first artificial intelligence agent trained by a method according to any one of the previously described embodiments. Hence, the advantages of the improved training methods are explicitly used for glycemic control of a patient.

The underlying problem is also solved by a computer implemented method of determining an insulin dose value for a patient next to administer and for administration by an insulin administration device based on a treatment algorithm comprising a first artificial intelligence agent (first AI agent), wherein the first AI agent is offline-trained based on in-silico data and real patient data sets, each forming a chronological continuous sequence, wherein at pre-defined time points the first AI agent determines a set of two or more than two consecutive insulin dose values, e.g. three consecutive insulin dose values, each two consecutive insulin dose values for administration in a pre-defined first time interval based on the weight of the patient and a present total daily dose value (TDD) of the patient as well as based on measured CGM data of the patient, administered insulin data and carbohydrate load data of the patient, wherein the measured CGM data, the administered insulin data and the carbohydrate load data are provided from measurements or other determination during a preceding pre-defined second time interval, wherein during determination of the set of insulin dose values the first AI agent optimizes the set of insulin dose values under a mandatory first constraint of a pre-defined limiting dose interval, wherein the first constraint applies to all insulin dose values of the determined set of insulin dose values, wherein the insulin dose value next to administer is determined from a sum of a carbohydrate load dependent bolus insulin dose value and of the first one of the determined set of insulin dose values.

The measured CGM data may be received from a continuous monitor directly or via a transmission device for and/or from the patient. In one embodiment, the administered insulin data may be received from an insulin administration device that is electrically connected with the control device realizing the above method.

In one embodiment, the set of two or more insulin dose values is a set of insulin dose values which is determined for administration to maintain proper blood glucose levels throughout the day that may be given, for example, continuously or pseudo-continuously (in first time intervals, e.g., every 1 to 20 minutes or every 5 hours) into the patient's subcutaneous tissue throughout the day to account for their basal insulin needs. In one embodiment, the insulin doses of each set are a dose of slow acting insulin (MDI). Alternatively, the set of two or more than two consecutive insulin dose values are fast or ultra-fast acting insulin to account for increased short term insulin needs and/or correct increased or rising blood glucose levels based on CGM values or predictions. Future, new insulin types might lead to adjustment of the insulin dose determination.

The prediction of the set of two or more than two consecutive insulin dose values is based on historical CGM data, administered insulin data, and, in one example, carbohydrate load data provided from a preceding pre-defined second time interval. The second time interval may be, e.g., from 12 hours to 2 hours. Further, the prediction is based on the actual weight of the patient that may be provided by an input of the patient. Additionally, the prediction is based on the present total daily dose (TDD). The TDD is a 24-hour insulin dose amount for dose determination using the first AI agent to stabilize glucose levels based on recent trends. In one embodiment, dose trends in non-postprandial periods are used to change TDD upwards or downwards. In one embodiment, the first AI agent may lock a stable TDD over time to avoid over-adjustment.

During determination of the set of insulin dose values the first AI agent optimizes the set of insulin dose values under a mandatory first constraint of a pre-defined limiting dose interval, wherein the first constraint applies to all insulin dose values of the determined set of insulin dose values. To determine not only the next insulin dose value to be administered but a set of at least two insulin dose values is advantageous because thereby outliers can be avoided. The first constraint may, e.g. comprise an adjustment-to-range constraint, for example, the prediction of the first AI agent that for each insulin value of the set of insulin dose values the related predicted CGM value is within a pre-defined first blood glucose range, e.g. within the range from 70 mg/dl to 180 mg/dl. In one embodiment, the optimization of the set of insulin dose values may be performed with regard to a second constraint. For example, the second constraint is a pre-defined minimum value of, for example, 54 mg/dl. A future CGM value predicted by the first AI agent for each one of the set of insulin dose values must not be fallen below this pre-defined minimum CGM value.

The insulin dose value next to administer is determined from a sum of a carbohydrate load dependent bolus insulin dose value and of the first one of the determined set of insulin dose values. The determined insulin dose value next to administer is provided at an output of the control device operating according to above method and may then be transmitted to an input of an insulin administration device such as an insulin pump or a smart pen for administration into the subcutaneous tissue of the patient. In one embodiment, the control device is integrally located in the insulin administration device. The carbohydrate load dependent bolus insulin dose value may, e.g., generally be determined based on user's input data of ingested carbohydrate load or on a carbohydrate estimation model, wherein the occurrence of carbohydrate intake is announced by the user, or on a meal prediction model, wherein the carbohydrate load and its occurrence is fully estimated without any user intervention.

According to an embodiment of the present invention, the present TDD is adjusted for a pre-defined third time interval based on a median of CGM data of the patient from the preceding time interval having the length of the third time interval during non-postprandial periods. The pre-defined third time interval may be from 12 hours to 5 hours. In one embodiment, the TDD may be adjusted by a pre-determined algorithm using pre-defined limits, wherein the TDD is adapted by means of at least one pre-defined factor and/or increase or decrease value if the median of CGM is within/above/below one/two of the pre-defined limits. The TDD adjustment is provided for further stabilization of glucose levels based on recent trends. In one embodiment, the system locks stable TDD values over time to avoid over-adjustment, for example, if the TDD value is unchanged over a pre-defined time period. A new adjustment period of TDD values may be re-started after another pre-defined period, e.g. after 30 hours, 60 hours or 100 hours.

In one embodiment for adjustment of the TDD a minimum margin and a maximum margin may be applied. In a further embodiment, an adaption of the minimum margin and/or the maximum margin is provided by an assessment of any one or several of the following parameters
- the outliers in the patient's CGM data of the preceding time interval,
- a time period below/above/within a pre-defined CGM value or interval,
- the median and/or standard deviation of CGM data of the patient from the preceding time interval having the length of the third time interval during non-postprandial periods.

The adjustment of the TDD minimum margin and maximum margin constitutes a confidence interval and therefor provides further user safety. The system considers safe boundaries based on previous dosing efficacy and hypo- or hyperglycemia occurrence. The adaption of the TDD minimum margin and the TDD maximum margin may be provided, in one embodiment, by a pre-determined algorithm using the above parameters, wherein the TDD maximum margin and the TDD minimum margin is adapted by means of at least one pre-defined factor and/or increase or decrease value if at least one of the above parameters fulfils the respective condition.

According to an embodiment of the present invention, the insulin dose value next to administer is determined by multiplying an aggressiveness factor to the first insulin dose value of the set of insulin dose values, wherein the aggressiveness factor is determined from outliers in the patient's CGM data in a fourth time interval and/or from an absolute value and/or from a change in patient's CGM data over the last pre-defined number of CGM data values. The fourth time interval may be from 10 minutes to 200 minutes. The pre-defined number of CGM data values may be from 10 to 2. For the absolute value of the CGM data and the change in CGM data pre-defined values/changes are provided. According to the comparison of the absolute value and/or the change in CGM data with the pre-defined values/changes, the factor is either 1 (i.e. first value of the set of insulin dose values is not changed) or greater than 1 or smaller than 1 (i.e. first value of the set of insulin dose values is changed). Accordingly, the insulin dose value next to administer is dynamically adjusted to provide safeguards for brittle or unstable patients reducing dose aggressiveness during rapid glucose declines or low CGM values. The adjustment may refer to a pre-defined past measured CGM values from the actual time point, e.g. the last 10 measured CGM values or less. For example, each 5 minutes a CGM measurement is provided by the CGM sensor. Dependent on the absolute value of the CGM measurement and its change within such short time interval, the aggressiveness factor is adjusted. The aggressiveness factor is, for example, between 0.6 and 1.5. Additionally, an instability score may be applied when adjusting the aggressiveness factor. The instability score measures the time points in which a hypoglycemia of a pre-defined severity is measured. E.g., if a CGM lower than 45 is measured, the instability score is incremented by 1. However, each increment has a time stamp so that after a pre-defined time interval after incrementation, e.g. 40 to 80 days, the increment is deleted from the instability score. The aggressiveness factor may be reduced based on the instability score, i.e. in case the actual instability score is above a pre-defined instability score limit of, e.g., 20.

According to an embodiment of the present invention, the carbohydrate load dependent bolus insulin dose value for the next meal is determined using an pre-defined algebraic meal prediction algorithm based on a current insulin-to-carb ratio (ICR), the CGM data of the patient of a fifth time interval from the time-point of the next meal, the carbohydrate load of the next meal (BW) and the present TDD. The meal prediction model may also be referred to as carbohydrate prediction model since it is based on the (pre-defined, predicted and/or announced) carbohydrate load of the next meal.

To cope with the glucose when the user ingests a meal and to avoid hyperglycemia, the carbohydrate load dependent bolus insulin dose value is provided. Due to delayed onset of current rapid-acting insulin formulations and the 5 to 15 minutes gab inherent to CGM measurement lag between blood glucoses values and interstitial glucose values measured in the interstitial space, the system provides a feed-forward action either from the user or inherently from the system such as meal announcement or meal prediction. Regarding meal announcement, it may come with the related carbohydrate load announcement or without, i.e. just announcing that meal ingestion is expected at a future time-point or is already in progress. The time-point at which for the user is estimated or announced that their next meal starts is referred to as the time-point of the next meal. The carbohydrate load dependent bolus may be determined when the meal is announced by the user, e.g. by a respective input, or immediately after a meal prediction.

According to above embodiment, the carbohydrate load dependent bolus insulin dose value for the next meal is calculated by a pre-defined algorithm based on the current insulin-to carb-ratio (ICR). For example, The ICR describes the coverage of a respective amount of glucose by one insulin unit. For example, the carbohydrate load dependent bolus insulin dose value is calculated considering a product of the carbohydrate load of the next meal (BW) and the ISR (i.e. BW x ICR). As the ICR may change for the respective user the ICR may be adapted as described below. However, the system starts with a pre-defined ICR for each patient depending on previous measurements. For the carbohydrate load dependent bolus insulin dose value further user-specific parameters, such as the weight of the user, may be considered.

Additionally, the CGM values of a fifth time interval just before the time-point of the next meal (i.e. a pre-prandial time interval) is considered to calculate the carbohydrate load dependent bolus insulin dose value in this embodiment. This is because the risk of hyperglycemia is high if the CGM values are already at a high level just before the patient begins their meal. For example, the fifth time interval may be the interval of 1 minute prior the next meal to the interval of 10 minutes prior the next meal. In one embodiment, an adaptive factor may be calculated depending on the CGM values measured within the fifth time interval. For example, the adaptive factor is lower then 1 if the CGM value (maximum or average of all measured CGM values of the fifth time interval) of the fifth time interval is lower than a pre-defined first CGM limit value, wherein in one embodiment the adaptive factor may vary dependent on the difference of the CGM value of the fifth time interval (maximum or average of all measured CGM values of the fifth time interval) and the pre-defined CGM limit value. In one example, the adaptive factor is greater than 1 if the CGM value of the fifth time interval (maximum or average of all measured CGM values of the fifth time interval) is lower than a second CGM limit value. In one embodiment, the adaptive factor is applied to the product of the carbohydrate load of the next meal (BW) and the ISR defined above.

The carbohydrate load of the next meal is the amount of carbohydrates as defined above but specified for the next meal. The present TDD and its variability is explained in detail above.

In one embodiment, the insulin on board is considered as a limit for administration of the calculated carbohydrate load dependent bolus insulin dose value for the next meal. The insulin on board refers to the insulin that is/will be active in the user's body in the future due to already and actually completed insulin administrated insulin doses. For example, the calculated carbohydrate load dependent bolus insulin dose value is only considered for the insulin dose value next to administer if the sum of (determined) administered insulin within the directly previous third time interval is lower than an insulin factor (InsF) times TDD. The insulin factor (InsF) is initially and continuously and/or dynamically be adapted, in particular based on historic time series data collected from a real or virtual CGM.

According to an embodiment of the present invention, the insulin-to-carb ratio (ICR) and/or the insulin factor (InsF) is/are adapted based on estimated post-prandial CGM data development of the patient in a post-prandial sixth time interval and for the carbohydrate load of the next meal.

In this embodiment the insulin-to-carb ratio (ICR) and/or the insulin factor (InsF) as explained above are adapted based on the development of the CGM values during a post-prandial sixth time interval which may cover between 2.5 hours and 5 hours directly after the time-point after the next meal. For example, the first artificial intelligence agent, another artificial intelligence agent different from the first artificial intelligence agent, or an algebraic algorithm may be used to predict the development of the CGM values during the post-prandial sixth time interval. According to the predicted development of the CGM values (e.g. if a carbohydrate load dependent bolus insulin dose value is not considered).

According to an embodiment of the present invention, for the actual carbohydrate load dependent bolus insulin dose value a push-down bolus is considered based on at least one of the following parameters: a temporal distance to a past number of closest meal time points, a time since last recorded meal, an estimated temporal distance to next meal, a pre-defined limit for the most recent CGM value of the patient, an estimated uncertainty and a time of the day.

In this embodiment, the push-down bolus shall adapt the carbohydrate load dependent bolus insulin dose value in a state in which the CGM value stays high but a meal is neither announced nor estimated, e.g. over a pre-defined past time period. Based on any one or a combination of above parameters, the system is configured to determine whether such push-down bolus is necessary. It may further determine which push-down bolus amount is needed. The push-down bolus is added to the actual carbohydrate load dependent bolus insulin dose value determined for an announced/estimated future meal. Accordingly, if there is no future meal announced/estimated, the push-down bolus forms the carbohydrate load dependent bolus insulin dose value and is considered for the insulin dose value next to administer.

According to an embodiment of the present invention, the carbohydrate load of the next meal is announced by the patient or estimated by a second artificial intelligence agent and/or wherein the meal time-point is a time point announced by the patient, e.g. by a single tab, or estimated by a third artificial intelligence agent.

This embodiment describes different mechanisms of carbohydrate load determination and meal time-point determination. In an easy but often unsafe manner the carbohydrate load and meal time-point may be announced by the user. However, many studies revealed that inputting by the patient is often erroneous. Accordingly, to make it more comfortable for the patient, in an alternative embodiment an automated estimation of meal carbohydrates, for example by AI modeling, is proposed. Instead of user-announced carbohydrate load, the second alternative provides the possibility for the user to announce the meal time-point of the next meal with a single push and the carbohydrate load of the next meal is estimated by, e.g., a second artificial intelligence agent. Of course, in a third alternative embodiment, both, the carbohydrate load of the next meal and the meal time-point may be estimated by the third artificial intelligence agent. Naturally, this is the most comfortable possibility for the patient. Some patients, however, may wish to have little more control regarding carbohydrate compensation and therefore may choose the first or second alternative.

Different embodiments of the second and third alternatives are described below.

The underlying problem is also solved by a computer implemented method of determining a carbohydrate load of the next meal based on a given next meal time-point , wherein the method comprises a second artificial intelligence agent, e.g. an LSTM-based sequential model, wherein the second artificial intelligence agent is trained to estimate a carbohydrate load to be received by the patient based on CGM data and meal period data of a pre-defined seventh time interval and further patient-related data and/or actual system-related input data, e.g. the patient weight and/or the actual TDD. For example, the next meal time-point can be given by the patient through a "single push" function. The patient preferably announces a meal by simply pressing a button on his electronic device and the computer-implemented method calculates the expected carbohydrate load based on their training.

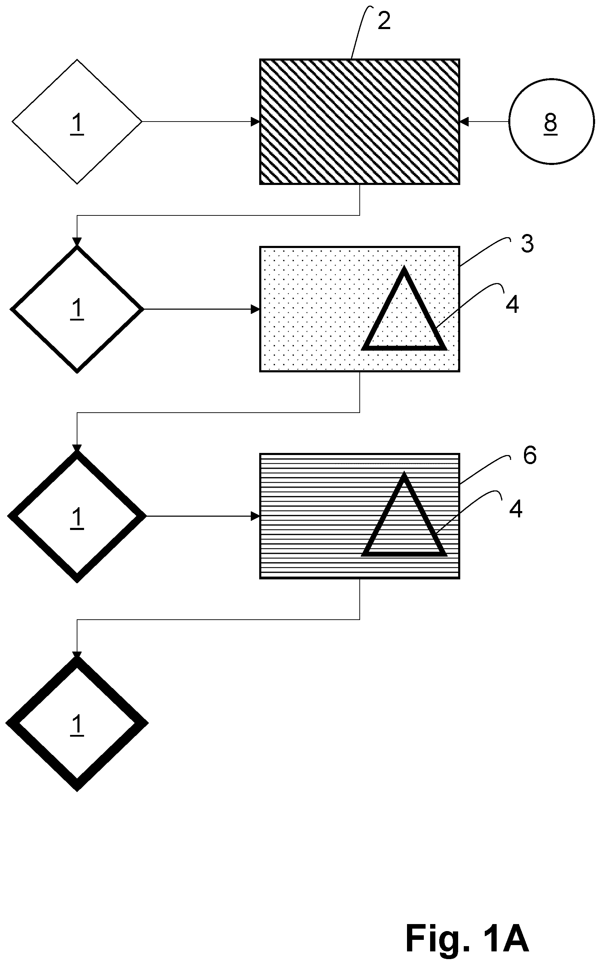

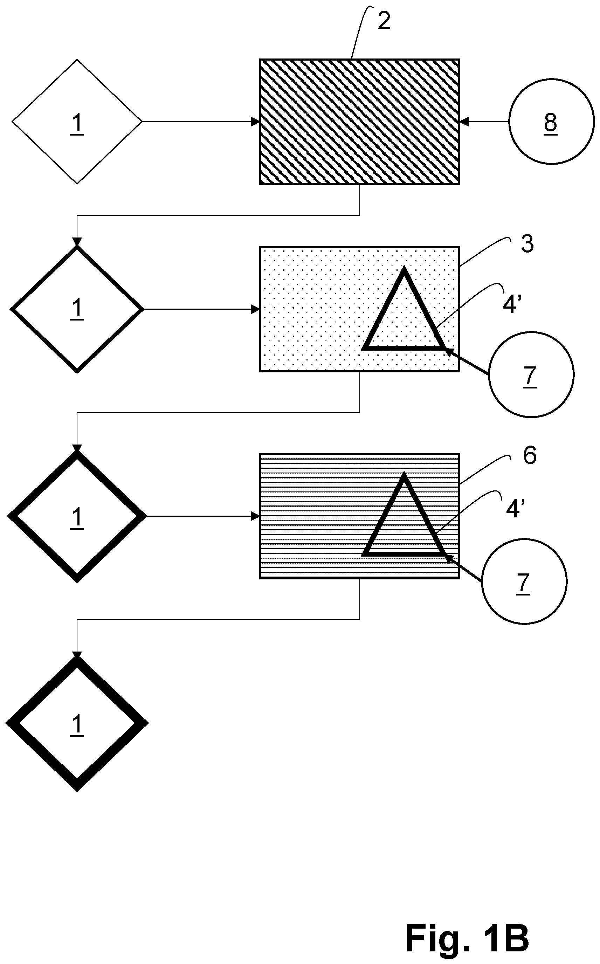

The second artificial intelligence agent can be a sequential artificial intelligence agent, in particular also a reinforcement learning agent, such that the training of the second AI agent is basically similar to the training of the first AI gent as described below in context of Figures 1A and 1B, or can be based on a Long Short-Term Memory (LSTM) based sequential model. The second Al agent is trained to predict a carbohydrate load on the basis of several input information such as blood glucose level and time of day, meal time, meal size, patterns and/or history of such data with the aim of optimizing the dosage of insulin.

An LSTM-based sequential model is a type of artificial neural network designed for processing and analyzing sequential data like CGM data. It is built upon Long Short-Term Memory (LSTM) units, which are a specialized form of recurrent neural networks (RNNs) capable of learning and retaining long-term dependencies within sequential datasets. The model incorporates a gating mechanism that regulates the flow of information through forget, input, and output gates, allowing it to selectively store, update, or discard information as needed. This architecture addresses issues such as vanishing or exploding gradients, enabling the reliable processing of time-dependent data.

In one embodiment the carbohydrate load dependent bolus insulin dose value for the patient for consideration for an insulin dose value next to administer by an insulin administration device may then be determined based on the estimated carbohydrate load, for example as indicated above.

In one embodiment, the second artificial intelligence agent is continuously adapted based on user-specific meal timing trends and postprandial CGM data of the patient.

Particularly, the second artificial intelligence agent is trained using patient's data over a pre-defined eighth time interval (calibration period).

The underlying problem is also solved by a computer implemented method of determining a carbohydrate load of a next meal of a patient and a time-point of this next meal comprising a third artificial intelligence agent, e.g. a sequential AI. For example, the third artificial intelligence agent is trained to estimate the meal time-point of the next meal of the patient by determining CGM trajectories and deviations indicative of a meal start within a pre-defined ninth time interval based on a pre-defined set of CGM data quantiles of a pre-defined tenth time interval and further patient-related data and/or system-related input data, e.g. the patient weight and/or the actual TDD.

The third artificial intelligence agent can be a sequential artificial intelligence agent, in particular also a reinforcement learning agent, such that the training of the second AI agent is basically similar to the training of the first AI gent as described below in context of Figures 1A and 1B, or can be based on a Long Short-Term Memory (LSTM) based sequential model as described above.

In one embodiment, the third AI agent may be continuously adapted based on user-specific meal timing trends and pre- and postprandial CGM data of the patient.

In one embodiment, the carbohydrate load dependent bolus insulin dose value for the patient for consideration for an insulin dose value next to administer by an insulin administration device is determined based on the estimated time-point and the carbohydrate load of the next meal of the patient, for example, as indicated above.

Particularly, the third AI agent is trained using patient's data over a pre-defined eighth time interval (calibration period).

Further features, advantages and examples of the invention can be found in the figures. These show:
- Figure 1A:: A schematic plan of a first exemplary course of a training method according to the invention.
- Figure 1B:: A schematic plan of a second exemplary course of a training method according to the invention.
- Figure 2:: A scheme illustrating the determination of the insulin dose value next to administer.
- Figure 3:: A scheme illustrating the determination of a carbohydrate load of the next meal of a patient.
- Figure 4:: A scheme illustrating the determination of a carbohydrate load and a time-point of the next meal of the patient.

Figure 1A schematically shows the course of a training method according to the invention. The training method shown here includes a basic training session 2, a simulator-based training session 3 and a refining training session 6.

At first the first Al agent 1 starts using an initial configuration (see first upper left rhombus "1" of Figure 1A) and is trained using the real patient data 8 (first training set of real patient data) in course of the basic training session 2. The real patient data 8 comprises real continuous blood glucose time sequences (CGM time sequences) and associated insulin dose values. Further, the real patient data 4 can comprise meal data and activity data. During each epoch of the basic training session 2, the first AI agent 1 runs through the complete first training set of real patient data 8. The first loss function of the basic training session 2 is configured such that the first AI agent 1 learns to mimic the insulin therapy, on which the real patient data 8 is based. This can be done by a first loss function which comprises a deviation norm for the real glucose blood values (from the real patient data) and the glucose blood values predicted by the first AI agent 1 and/or a deviation norm for the real insulin dose values (from the real patient data) and insulin dose values determined by the first AI agent 1. The basic training session 2 ends when a convergence criterion is fulfilled, for example if further training would not enhance the first AI agent's precision (the loss function extrema) anymore in a pre-defined way. It can be said that after the basic training session 2, the first AI agent 1 is as good in controlling a patient's glycemic behavior as the real therapy actors, on which the real patient data 8 are based. From the basic training session 2 a pre-trained first AI agent 1 results (see second upper left rhombus "1" of Figure 1A).

After the basic training session 2, the first AI agent 1 - which is, accordingly, used in a pre-trained state - is trained in course of the simulator-based training session 3. During each epoch of the simulator-based training session 3, the first AI agent 1 runs through different glycemic scenarios. These scenarios vary amongst others in the underlying patient type (e.g. age or sex), the glucose start values as well as the underlying meal or activity situation. During each epoch, a full society population can be covered by these scenarios. The first AI agent 1 is trained by using simulated feedback data in this session. In the shown case, a pre-defined metabolic simulator 4 is used, which is based on heuristic equations or data-driven grey-box identification. During the simulator-based training session 3, the first AI agent 1 receives a starting blood glucose time series (CGM values) and background data like age and sex of the patient. Then, the first AI agent 1 determines an insulin dose value next to administer based on the received data. The pre-defined metabolic simulator 4 determines then the metabolic response of the patient's blood glucose system (feedback) to this insulin dose value, updating the starting blood glucose time series. Then, the first AI agent 1 determines an insulin does value again. This goes back and forth, wherein events can occur like meal or activity events depending on the simulated scenario. The first AI agent 1 is rewarded if the simulated blood glucose value (CGM value) stays within a predefined range. The first AI agent 1 is penalized if the simulated blood glucose value (CGM value) is outside the pre-defined range, especially if the simulated blood glucose value reaches values which are dangerous to life or even lethally in reality. Hence, the simulator-based training session 3 allows the first AI agent 1 to try out different therapies and to find out the best therapy in terms of pre-defined desired blood glucose range to stay in.

After the simulator-based training session 3, the first AI agent 1 is very well trained. This training state is represented in Figure 1A by the second last rhombus "1" on the left hand side. Nevertheless, the training of the first AI agent 1 can be continued as shown in Figure 1A by using a refining training session 6, which can be based on heuristic models and - in contrast to the basic training session 2 and the simulator-based training session 3 - is not necessarily a machine learning training. For example, during the refining training session 6, the first AI agent 1 is taught to keep pre-defined dosing thresholds or the keep other heuristically pre-defined constraints such as blood glucose ranges. Additionally, or alternatively, the pre-defined metabolic simulator 4 used for the simulator-based training session 3 or another metabolic simulator can also be used for the refining training session 6 in a way that the impact of policies to be applied is validated by using the respective pre-defined metabolic simulator 4. The result is a further trained first AI agent represented by the last rhombus "1" on the left hand side of Figure 1A.

Figure 1B shows a second example for a training method according to the present invention. This training method is very similar to the one described above and shown in Figure 1A. Indeed, the training method shown in Figure 1B differs from the training method shown in Figure 1A only with respect to the used simulator. In contrast to a pre-defined metabolic simulator 4, which is used in the example of Figure 1A, a data-driven metabolic simulator 4' is used in the example of Figure 1B. The data-driven metabolic simulator 4' is based on a second training data set 7 of real patient data. In contrast to the pre-defined metabolic simulator 4, the output of the data-driven metabolic simulator 4' is not completely heuristically defined by fixed equations. Rather, the output of the data-driven metabolic simulator 4' is a product of machine learning techniques applied to the second training set 7 of real patient data.

The first AI agent 1 in the examples of Figures 1A and 1B is a reinforcement learning agent. During training epochs such the first AI agent 1 can learn, e.g., more than 50 million parameters, i.e. structural features, which enables a high level of prediction accuracy for later insulin dosing.

In the following, the principle of reinforcement learning is briefly summarized in view of the present invention. The goal of reinforcement learning is to train the first AI agent 1 that can take actions, namely dosing determination, based on observations and rewards from the environment, e.g. sensorial CGM data and administered insulin data, in order to achieve a defined goal, here: keep the blood glucose in a predefined range. In course of an epoch training, the first AI agent receives an observation and a reward from the environment (real data during the basic training session or simulator feedback during the simulator-based training session) at each time step and sends an action back. The reward indicates how successful the first AI agent's previous action was in relation to the task goal. The agent and environment interact in discrete time steps, where the first AI agent makes a decision and receives feedback from the environment. The first AI agent's observations can be divided into multiple channels, each containing elements from either a continuous numerical set or a finite discrete set. The action is a one-dimensional input, while the reward is a numeric scalar. In context of a reinforcement learning, the first AI agent 1 comprises main components: a policy and a learning algorithm.

The first AI agent's policy is a mapping from the current environment observation to a probability distribution over possible actions. This policy is implemented using a function approximator with tunable parameters, such as a deep neural network. The learning algorithm continuously updates the policy's parameters based on the actions, observations, and rewards received, aiming to find an optimal policy that maximizes the expected cumulative long-term reward.

The learning algorithm can work with one or more parameterized function approximators that learn the policy. These approximators are categorized into two main types: critics and actors. A critic estimates the value of the policy (the expected cumulative long-term reward) for a given observation and action. An actor, on the other hand, selects the action that maximizes the policy value for a given observation.

In the shown example, the first AI agent uses both actors and critics. In such agents, during training, the actor learns the best action to take using feedback from the critic, rather than directly using the reward. Simultaneously, the critic learns the value function from the rewards to provide proper feedback to the actor.

Preferably, the method of Twin-Delayed Deep Deterministic Policy Gradient is used to train the first Al agent 1 (here: reinforcement learning agent). The Twin-Delayed Deep Deterministic Policy Gradient (TD3) algorithm is an off-policy actor-critic method for continuous action spaces that improves the Deep Deterministic Policy Gradient (DDPG) algorithm. TD3 reduces value function overestimation by using two Q-value critics and selecting the minimum estimate for policy updates. The algorithm also delays updates to the policy and target networks, improving stability during training. Additionally, noise is added to the target action to prevent exploitation of high Q-value estimates. TD3 can be trained with two critics (full TD3) or a single critic (delayed DDPG with target policy smoothing). The training process involves updating the actor and critics using mini-batches sampled from an experience buffer. Target networks are updated periodically, either with or without smoothing, to stabilize learning.

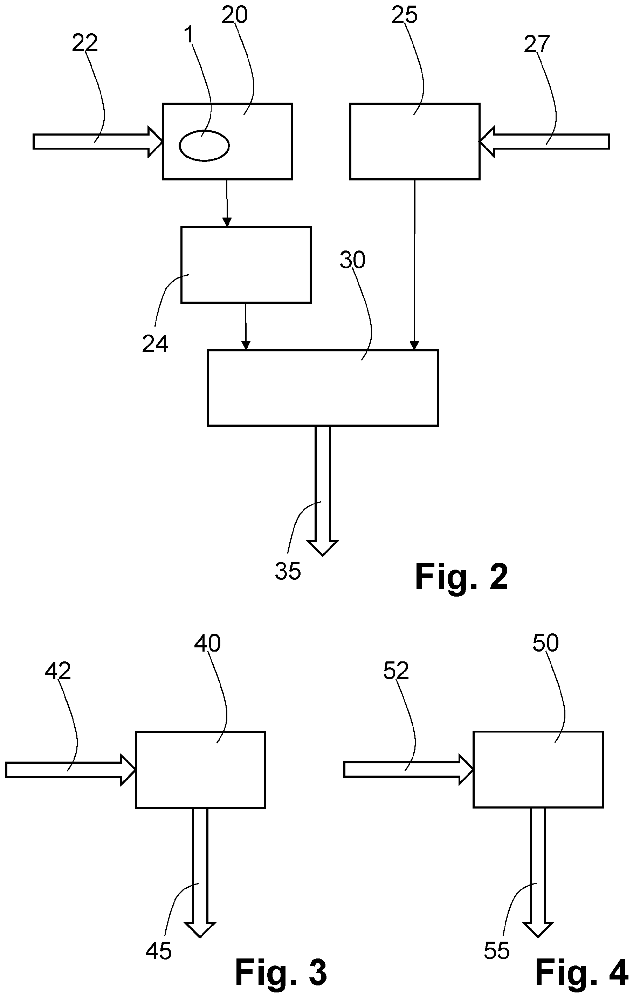

The application of the first AI agent 1 trained as indicated above for determination of the insulin dose value next to administer by an insulin administration device formed by an insulin pump is described in the following using the scheme of Figure 2. Analogously, such determination may be provided for a smart pen but with a greater time period between single administration steps.

In step 20 the first AI agent 1 determines a set of three consecutive insulin dose values each for administration at time intervals of 5 minutes. The first AI agent 1 determines this set of insulin dose values based on the present TDD, the CGM data of the last 5 hours and the administered insulin data of the last 5 hours. These data are received by the first AI agent 1 as input parameters (see arrow 22 in Figure 2). In one example, additionally carbohydrate load data from a previous time interval may be provided as an input parameter. The TDD may be adjusted during usage of the system as indicated above. Additionally, the TDD margins for TDD adjustment may be adjusted, as well, as explained above.

In step 20 the first AI agent 1 optimizes this set of insulin dose values such that the related predicted CGM value is within a pre-defined first blood glucose range, e.g. within the range from 70 mg/dl to 180 mg/dl. In one embodiment, the optimization of the set of insulin dose values may be performed with regard to a second constraint being that a future CGM value predicted by the first Al agent for each one of the set of insulin dose values must not be fallen below a minimum CGM value of, for example, 54 mg/dl.

In the next step 24, the first one of the set of three insulin dose values is multiplied by an actual aggressiveness factor. This aggressiveness factor is 1 if the last four measured CGM data is within a pre-defined range of, e.g., 90 to 170 mg/dl. If one of the last four measured CGM data is below 90 mg/dl the aggressiveness factor is lowered down to 0.7 and if one of the last four measured CGM data is above 170 mg/dl the aggressiveness factor is raised up to 1.6.

As indicated above, the aggressiveness factor may be dynamically adjusted, e.g. based on the last measured 10 CGM measurement values and the instability score.

In step 25 a carbohydrate load dependent bolus insulin dose value (in the following short: meal bolus) is determined based on the carbohydrate load data (BW) and the time-point of the next meal. It may be determined when the meal is announced by the user, e.g. by a respective input or immediately after the meal is predicted. Further, the CGM data of a pre-defined time interval, e.g. the last 3 hours prior the time-point of the next meal, the actual ICR, the insulin factor (InsF), the weight of the patient and the actual TDD are provided as additional input parameters (see arrow 27). The carbohydrate load (BW) and the time-point of the next meal may be announced by the patient or estimated as indicated below. The ICR and/or the InsF may be adapted as indicated above.

In particular, the meal bolus is calculated according to the following formula: $Meal bolus = adaptive factor / \left(BW\times ICR\right)$

The adaptive factor is a factor determined from the CGM values of a short time period just shortly before the time-point of the next meal, e.g. 3 minutes. As indicated above, it may adapt the meal bolus to the respective measured CGM values.

Further, in one embodiment, if a meal bolus calculated as explained above is not applicable, a push-down bolus may be determined in step 25. The push-down bolus is determined, for example, by averaging the additional insulin injected to counteract previous meals within the same time frame in the last 5 days, injected gradually over time, 3 hours before the mean time of meal within the same time window in the last 5 days.

Additionally, as indicated above, a meal bolus (push-down bolus) determined as indicated above may be reduced to a lower value or zero if the product of the insulin factor (InsF) of the patient and the actual TDD is greater than the insulin amount administered during a time interval of the previous 9 hours from the actual time point. The reduced meal/push-down bolus is therefore calculated based on the 9 hour windowed insulin limit and the currently active insulin on board based on the total dose injected in the past 9 hours.

In step 30 the, if applicable, reduced meal / push-down bolus determined in step 25 is added to the insulin bolus determined in step 24. The sum insulin value is provided at the output of the computational unit (see arrow 35 in Figure 2) and transmitted to the pump unit to deliver the respective short-acting insulin dose to the patient.

For the next dose, for example after 5 minutes, the above method is repeated.

In one embodiment, the patient may announce the carbohydrate load of the next meal and the time-point of the next meal using an input unit, e.g. a touch pad.

Alternatively, the patient may just announce the time-point of the next meal by a single push at the touch pad. As depicted in Figure 3, triggered by this announcement (see arrow 42), a second artificial intelligence agent may determine a respective carbohydrate load in step 40 and provide this carbohydrate load at its interface (see arrow 45 in Figure 3) to step 25 as an input parameter (arrow 27) for this step.

The second artificial intelligence agent may be a Long Short-Term Memory (LSTM) based sequential model. This model is trained with real patient data to estimate the carbohydrate load based at least on the time of the single push announcement and the previously measured CGM data. Such an LSTM model possesses a gate structure that fits to such an underlying problem. A LSTM is a type of recurrent neural network (RNN) specifically designed to handle sequential data and overcome the vanishing gradient problem often encountered in traditional RNNs. It excels at learning long-term dependencies by utilizing a cell structure composed of three key gates: input gate, forget gate, and output gate. The input gate decides which new information should be added to the cell state, the forget gate determines what information should be discarded, and the output gate regulates the output based on the updated cell state. This gated architecture allows LSTMs to selectively retain or discard information, enabling them to maintain context over long sequences. Hence, it effectively processes the task of predicting the carbohydrate load based on the given input parameters such as day time and previous CGM data. During training, the LSTM based agent update its internal weights through backpropagation and gradient descent, ensuring it adapt to patterns in the sequential input data while mitigating the loss of information over time. This makes the LSTM based agent highly suitable for capturing both short-term patterns and long-term dependencies within the real patient data.

As a further alternative, as depicted in Figure 4, triggered by changes or trends in the CGM time series data (arrow 52), a third artificial intelligence agent may determine a carbohydrate load and a time-point for the next meal in step 50 and provide this carbohydrate load and this time-point at its interface (see arrow 55 in Figure 4) to step 25 as an input parameter (arrow 27) for this step.

As the second artificial agent, the third artificial intelligence agent predicts carbohydrate load based on previous CGM data time series, but without the additional information of the specific time of the expected carbohydrate load - such that no push announcement is necessary anymore. It turned out, that also for the third artificial intelligence a LSTM based agent can be used.

## Claims

1. A computer implemented training method for training a first artificial intelligence agent for glycemic control of a patient,
wherein the training method comprises a basic training session comprising one or more training epochs,
wherein a first training data set of real patient data is used as input data for the basic training session, such that in course of the basic training session the first AI agent receives real-patient chronological continuous data sequences and is trained to control the glycemic behavior of the patient based on these real-patient chronological continuous data sequences,
wherein the training method comprises additionally a simulator-based training session comprising one or more training epochs,
wherein during a training epoch of the simulator-based training session, particularly each training epoch of the simulator-based training session, the first AI agent is trained to control the glycemic behavior of the patient based on glycemic feedback data of simulated patients, wherein the glycemic feedback data is generated by a metabolic simulator.

2. The training method of claim 1,
wherein during the basic training session, a first loss function is used, wherein the first loss function comprises
- a deviation norm for a deviation between a glycemic behavior prediction of the first AI agent and the glycemic behavior according to the first training data set of real patient data and/or
- a deviation norm for a deviation between insulin dose values to be administered determined by the first AI agent and insulin dose values administered according to the first training data set of real patient data,
wherein during the simulator-based training session, a second loss function is used, wherein the second loss function
- a deviation norm for the deviation between a glycemic behavior driven by insulin dose values, which are determined by the first AI agent, and a predefined optimal range of the glycemic behavior of simulated patients.

3. The training method of any one of the previous claims,
wherein during a training epoch of the simulator-based training session, particularly each training epoch of the simulator-based training session, the first AI agent is confronted with several different simulated glycemic scenarios, which differ, for example, with respect to a simulated patient of the underlying patient type characterized, for example, by its age and sex, wherein for each scenario the following steps are conducted using a metabolic simulator, wherein steps c) and d) are repeated multiple times:
a) The first AI agent receives initial simulated patient data from the metabolic simulator;
b) The first AI agent determines an insulin dose value next to administer based on the initial simulated patient data received in step a);
c) The metabolic simulator determines the simulated patient's glycemic behavior as glycemic feedback data based on the insulin dose value determined in step b) as if the directly previously determined insulin dose value had been administered to the simulated patient;
d) The first AI agent determines an insulin dose value next to administer based on the feedback data determined in step c) and continues with step c) using the determined insulin dose value next to administer in step d).

4. The training method of any one of the previous claims, wherein according to a fist alternative, the metabolic simulator of the simulator-based training session is a data-driven metabolic simulator, wherein the feedback data generated by the data-driven metabolic simulator is determined in dependence on real or simulated patient data, wherein the data-driven metabolic simulator is e.g. a grey-box identification simulator,
wherein according to a second alternative the metabolic simulator of the simulator-based training session is a pre-defined metabolic simulator, e.g. a non-linear system identification simulator.

5. The training method of any one of the previous claims, wherein the first training data set of real patient data comprise Continuous Glucose Monitoring (CGM) data, administered insulin data and carbohydrate load data, each data having a time stamp.

6. The training method of any one of the previous claims, wherein the first Al agent is a reinforcement learning agent, preferably a Twin-Delayed Deep Deterministic Policy Gradient agent, such that a training epoch of the first Al agent is performed by the interaction of an actor, for example an actor neuronal network, and at least one critic, for example a critic neuronal network.

7. A computer implemented method of determining an insulin dose value for a patient next to administer and for administration by an insulin administration device comprising the usage the first artificial intelligence agent trained by a training method according to any one of the previous claims.

8. A computer implemented method of determining an insulin dose value for a patient next to administer and for administration by an insulin administration device based on a treatment algorithm comprising a first artificial intelligence agent, wherein the first AI agent is offline-trained based on in-silico data and real patient data sets , each forming a chronological continuous sequence, wherein at pre-defined time points the first AI agent determines a set of two or more than two consecutive insulin dose values, e.g. three consecutive insulin dose values, each two consecutive insulin dose values for administration in a pre-defined first time interval based on the weight of the patient and a present total daily dose (TDD) of the patient as well as based on measured CGM data of the patient, administered insulin data and carbohydrate load data of the patient, wherein the measured CGM data, the administered insulin data and the carbohydrate load data are provided from measurements or other determination during a preceding pre-defined second time interval, wherein during determination of the set of insulin dose values the first AI agent optimizes the set of insulin dose values under a mandatory first constraint of a pre-defined limiting dose interval, wherein the first constraint applies to all insulin dose values of the determined set of insulin dose values, wherein the insulin dose value next to administer is determined from a sum of a carbohydrate load dependent bolus insulin dose value and of the first one of the determined set of insulin dose values.

9. The method of claim 8, wherein the present insulin-per-day limit is adjusted for a pre-defined third time interval based on a median of CGM data of the patient from the preceding time interval having the length of the third time interval during non-postprandial periods.

10. The method of any one of the claims 7 to 8, wherein insulin dose value next to administer is determined by multiplying an aggressiveness factor to the sum of an carbohydrate load dependent carbohydrate load dependent bolus insulin dose value and of the first one of the determined set of insulin dose values, wherein the aggressiveness factor is determined from outliers in the patient's CGM data in a fourth time interval and/or from an absolute value and/or from a change in patient's CGM data over the last pre-defined number of CGM data values.

11. The method of any one of the claims 8 to 10, wherein the carbohydrate load dependent bolus insulin dose value for the next meal is determined based on a current insulin-to-carb ratio, the CGM data of the patient of a fifth time interval from the time-point of the next meal, the carbohydrate load of the next meal and the present TDD.

12. The method of any one of the claims 10 or 11, wherein for the actual carbohydrate load dependent bolus insulin dose value a push-down bolus is considered based on at least one of the following parameters: a temporal distance to a past number of closest meal time points, a time since last recorded meal, an estimated temporal distance to next meal, a pre-defined limit for the most recent CGM value of the patient, an estimated uncertainty and a time of the day.

13. The method of any one of the claims 8 to 12, wherein the carbohydrate load of the next meal is announced by the patient or estimated by a second artificial intelligence agent and/or wherein the meal time-point is a time point announced by the patient, e.g. by a single tab, or estimated by a third artificial intelligence agent.

14. A computer implemented method of determining a carbohydrate load of the next meal based on a given next meal time-point, wherein the method comprises a second artificial intelligence agent, wherein the second artificial intelligence agent is trained to estimate a carbohydrate load to be received by the patient based on CGM data and meal period data of a pre-defined seventh time interval.

15. A computer implemented method of determining a carbohydrate load of a next meal of a patient and a time-point of this next meal comprising a third artificial intelligence agent, wherein the third artificial intelligence agent is trained to estimate the meal time-point and the carbohydrate load of the next meal of the patient by determining CGM trajectories and deviations indicative of a meal within a pre-defined ninth time interval.
